(19) Europäisches Patentamt
European Patent Office
Office européen des brevets

(11) Veröffentlichungsnummer: **0 282 760 B1**

(12) **EUROPÄISCHE PATENTSCHRIFT**

(45) Veröffentlichungstag der Patentschrift: **17.06.92**

(51) Int. Cl.⁵: **C07C 403/08**, C07C 403/14, C07C 47/21, C07C 47/277, C07D 319/06, C07C 69/73, C07C 45/38

(21) Anmeldenummer: **88102519.1**

(22) Anmeldetag: **20.02.88**

(54) **Verfahren zur Herstellung von Polyenaldehyden.**

(30) Priorität: **24.02.87 DE 3705785**

(43) Veröffentlichungstag der Anmeldung:
**21.09.88 Patentblatt 88/38**

(45) Bekanntmachung des Hinweises auf die Patenterteilung:
**17.06.92 Patentblatt 92/25**

(84) Benannte Vertragsstaaten:
**CH DE FR GB LI**

(56) Entgegenhaltungen:

**J. AM. CHEM. SOC., Band 106, 1984, Seiten 3374-3376, American Chemical Society; M.F. SEMMELHACK et al.: "Oxidation of alcohols to aldehydes with oxygen and cupric ion, mediated by nitrosonium ion"**

**TETRAHEDRON, Band 36, Nr. 9, 1980, Seiten 1191-1194, Pergamon Press Ltd., Oxford, GB; C. JALLABERT et al.: "Déshydrogénation d'alcools en composés carbonylés par le système CuCl/O2/Ligande"**

**ANGEWANDTE CHEMIE, Band 18, Nr. 11, November 1979, Seiten 864-865, Verlag Chemie, GmbH, Weinheim, DE; C. BERTI et al.: "Enantioselective oxidation by a chiral acylaminyl oxide"**

(73) Patentinhaber: **BASF Aktiengesellschaft**
**Carl-Bosch-Strasse 38**
**W-6700 Ludwigshafen(DE)**

(72) Erfinder: **Knaus, Günter H., Dr.**
**Wanderstrasse 37**
**W-6700 Ludwigshafen(DE)**
Erfinder: **Paust, Joachim, Dr.**
**Ringstrasse 3**
**W-6708 Neuhofen(DE)**

EP 0 282 760 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Polyenaldehyden, insbesondere von Vitamin-A-Aldehyd.

Die Überführung eines Polyenalkohols in einen Polyenaldehyd ist insofern keine triviale Reaktion, als das umzusetzende Substrat eine sehr empfindliche Substanz darstellt. Wie in Synthesis 1976, S. 65ff, insbesondere S. 74 dargelegt, können folglich nur wenige der zur Oxidation von $\alpha,\beta$-ungesättigten Alkoholen geeigneten Verbindungen zur Darstellung von Polyenaldehyden herangezogen werden.

Die Fähigkeit eines Oxidationsmittels, empfindliche Polyenalkohole wie beispielsweise Vitamin-A-, Vitamin-A-Derivate und Carotinoide ohne Isomerisierungen zu den Aldehyden zu oxidieren, wird oft zur Charakterisierung der besonders milden Arbeitsweise dieses Reagenzes verwendet, vgl. Synthesis 1976, S. 65ff, insbesondere S. 75 und Houben-Weyl VII/1, S. 178 (1954). Als geeignete Reagenzien zur Oxidation von Polyenalkoholen zu den entsprechenden Aldehyden haben sich vor allem Metalloxide wie Mangandioxid oder Nickelperoxid erwiesen.

Unglücklicherweise können diese Oxidationsmittel allerdings nur heterogen eingesetzt werden, so daß ihre Oxidationseigenschaften stark von der Verteilung und der Oberflächenbeschaffenheit des eingesetzten Oxids abhängen. Folglich können die Ausbeuten von Ansatz zu Ansatz stark schwanken (Chem.Rev. 67, 188, 1967). Entsprechend aktive Metalloxide müssen zudem nach speziellen Verfahren hergestellt werden, vgl. DE-A-25 29 605 und DE-A-24 15 928. Als weiterer Nachteil der Metalloxide muß angeführt werden, daß sie meist in großem Überschuß (vgl. J. Chem. Soc. (1952), 1094), mindestens aber äquimolar eingesetzt werden müssen und zudem die Reaktionszeiten recht lang sein können.

Ein Verfahren zur Herstellung von Retinal mit den Merkmalen des Oberbegriffts des Anspruchs 1 ist in der CH-A-337 500 beschrieben. Die Oxidation ist platinkatalysiert. Nachteilig an diesem Verfahren ist die Tatsache, daß dieses unwirtschaftlich ist. Man muß relativ große Mengen an Platinkatalysatoren einsetzen, und die erzielten Ausbeuten sind trotzdem unbefriedigend. Dies dürfte nicht zuletzt darauf zurückzuführen sein, daß im Gerüst des Retinols zahlreiche oxidationsempfindliche Doppelbindungen und Allylstellungen vorhanden sind. Deshalb wird beim Umgang mit Polyenen ganz allgemein zum Arbeiten unter Sauerstoffausschluß geraten, vgl. Houben-Weyl 5/1d. Seite 13 (1972).

Es ist bekannt, daß das Katalysatorsystem 2,2,6,6-Tetramethylpiperidin-1-oxyl und Kupfer(I)chlorid zur Oxidation allylischer Alkohole herangezogen werden kann, vgl. JACS 106, 3374-3376 (1984). Man mußte jedoch damit rechnen, daß bei der Anwendung dieses Katalysatorsystems auf empfindliche Polyenalkohole Isomerisierungen auftreten und die Ausbeuten an Wertprodukt wesentlich vermindert werden.

Der Erfindung liegt die Aufgabe zugrunde, ein Verfahren zur Herstellung von Polyenaldehyden der allgemeinen Formel I

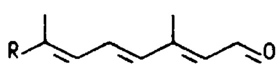 (I)

worin R für

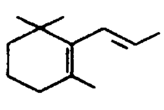 , 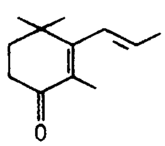 , 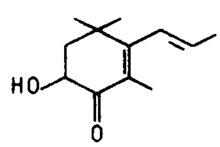 , 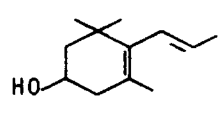 ,

-COOR' , -CHO oder

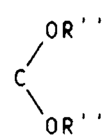

steht

wobei R′ die Bedeutung Alkyl, Aryl, Aralkyl, Cycloalkyl besitzt und gewünschtenfalls weiter substituiert sein kann und die Reste R″ für $C_1$-$C_4$-Alkyl stehen oder gemeinsam $-CH_2-CH_2-$ oder $-CH_2-CH_2-CH_2-$Brücken bedeuten, die gewünschtenfalls durch $C_1$-$C_4$-Alkyl weiter substituiert sein können, durch katalytische Oxidation des entsprechenden Polyenalkohols mit Sauerstoff oder einem sauerstoffhaltigen Gas, so auszugestalten, daß man die empfindlichen Polyenalkohole ohne nennenswerte Isomerisierung in vorzüglichen Ausbeuten oxidieren kann.

Diese Aufgabe wird gelöst mit einem Verfahren der genannten Art, das dadurch gekennzeichnet ist, daß man die Oxidation in Gegenwart des Katalysatorsystems 2,2,6,6-Tetramethylpiperidin-1-oxyl oder 4-Oxo-2,2,6,6-tetramethylpiperidin-1-oxyl und Kupfer(I)chlorid durchführt.

Nach einer bevorzugten Ausführungsform des erfindungsgemäßen Verfahrens stellt man aus (all-E)-Retinol das (all-E)-Retinal her.

Nach einer besonders zweckmäßigen Ausführungsform des erfindungsgemäßen Verfahrens arbeitet man in homogener Phase in einem Lösungsmittel.

Als Lösungsmittel kommen insbesondere organische Lösungsmittel in Betracht. Besonders bevorzugt sind N,N-Dimethylformamid, N,N-Dimethylacetamid, 1-Methyl-2-pyrrolidon, N,N-Dimethylpropylenharnstoff oder N,N-Dimethylethylenharnstoff.

Nach einer besonders vorteilhaften Ausgestaltung des erfindungsgemäßen Verfahrens setzt man die Katalysatorkomponente 2,2,6,6-Tetramethylpiperidin-1-oxyl oder 4-Oxo-2,2,6,6-tetramethylpiperidin-1-oxyl und Kupfer(I)chlorid im molaren Verhältnis von 1:0,5 bis 1:3 ein.

Nach einer weiteren zweckmäßigen Ausgestaltung der Erfindung werden die einzelnen Katalysatorkomponenten jeweils in einem Anteil von 1 bis 10 Mol%, insbesondere 5 bis 10 Mol%, bezogen auf den eingesetzten Polyenalkohol, verwendet.

Die nach dem erfindungsgemäßen Verfahren herstellbaren Polyenaldehyde sind entweder, wie Vitamin-A-Aldehyd, Verbindungen mit wertvoller biologischer Aktivität, oder dienen als Schlüsselverbindungen zur Darstellung wichtiger Carotinoide. So kann aus Retinal durch Wittig-Reaktion mit Triphenyl(retinyl)-phosphoniumsalzen das begehrte $\beta$-Carotin hergestellt werden (vgl. DE-C-1 068 709). Ausgehend von Retinal sind durch Wittig-Reaktionen mit (1,1-Dimethoxy-3-methyl-2-butenyl)triphenylphosphoniumchlorid zudem die $\beta$-Apocarotinale zugänglich (vgl. Lieb. Ann. Chem. 1976, 2194-2205). Der 2,6-Dimethyl-8-oxo-octa-2,4,6-trien-1-säureethylester läßt sich mit Triphenyl(retinyl)phosphoniumchlorid bequem zum begehrten Lebensmittelfarbstoff $\beta$-Apo-8′-carotinsäureethylester umsetzen, vgl. Helv. Chim. Acta, 49, 369, 1966.

Man kann anstelle der Katalysatorkomponente 2,2,6,6-Tetramethylpiperidin-1-oxylauch das 4-Oxo-2,2,6,6-tetramethylpiperidin-1-oxyl einsetzen, wobei man dieselben Ergebnisse erzielt. Das ist insofern vorteilhaft, als diese Verbindung in nur 2-Stufen aus Ammoniak und Aceton zugänglich ist. Hierdurch ist das Verfahren noch wirtschaftlicher.

Zur praktischen Durchführung der Reaktion wird Sauerstoff oder ein sauerstoffhaltiges Gas durch eine mit dem Katalysatorsystem versetzte Lösung der Substanz in einem der genannten Lösungsmittel geleitet. Die Reaktionstemperatur beträgt zweckmäßig 0 bis 40 °C, vorzugsweise 20 bis 40 °C.

Nach Beendigung der Reaktion kann die Wertsubstanz chromatographisch vom Katalysator getrennt werden, was bei dem eingesetzten 4-Oxo-2,2,6,6-tetramethylpiperidin-1-oxyl auf Grund seines niedrigen Rf-Wertes noch besser als beim 2,2,6,6-Tetramethylpiperidin-1-oxyl geht. Eine andere Aufarbeitungsvariante besteht darin, die Wertsubstanz durch ein geeignetes Lösungsmittel aus der mit Wasser bzw. wässriger Kochsalzlösung verdünnten Reaktionslösung zu extrahieren und Restmengen an Katalysator durch Schütteln mit einer Lösung von Natriumjodid in Salzsäure zu entfernen. Die so erhaltene Rohsubstanz kann dann durch Kristallisation oder Chromatographie gereinigt werden.

In der allgemeinen Formel I kann der Rest R′ die Bedeutungen Alkyl, Aryl, Aralkyl oder Cycloalkyl besitzen. Gewünschtenfalls kann der Rest R′ noch weiter substituiert sein. Unter Alkyl ist insbesondere $C_1$-$C_{20}$-Alkyl, besonders bevorzugt $C_1$-$C_6$-Alkyl zu verstehen. Aryl umfaßt insbesondere Phenyl und Naphthyl. Unter Aralkylresten sind beispielsweise ein $C_1$-$C_6$-Phenylrest, insbesondere der Benzylrest zu verstehen. Beim Cycloalkylrest kann es sich beispielsweise um einen $C_3$-$C_{12}$-Cycloalkylrest handeln. Soweit die Reste R′ weiter substituiert sind, kann es sich bei den Substituenten um $C_1$-$C_6$-Alkoxyreste, OH, Halogen, und gegebenenfalls substituiertes Amino handeln.

Die Reste R″ können für $C_1$-$C_4$-Alkyl, insbesondere Methyl, stehen. Sie können gleich oder verschieden sein. Die Reste R″ können aber auch $-CH_2-CH_2-$ oder $-CH_2-CH_2-CH_2-$Brücken darstellen und so ein cyclisches Acetal bilden. Die Brücken können gewünschtenfalls durch $C_1$-$C_4$-Alkylreste mono- oder disubstituiert sein. Ein Beispiel ist der Rest

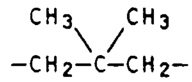

der die folgende Acetalgruppierung

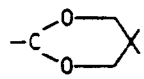

ergibt.

Wenn man als Oxidationsmittel ein sauerstoffhaltiges Gas einsetzt, handelt es sich insbesondere um Luft oder um Gasgemische, welche mit Sauerstoff versetzt bzw. angereichert wurden.

Die Erfindung wird durch die nachfolgenden Beispiele weiter erläutert.

Beispiel 1

Oxidation von Retinol zu Retinal unter Verwendung von 2,2,6,6-Tetramethylpiperidin-1-oxyl/Kupfer(I)chlorid als Katalysator

Durch eine Lösung von 10,0 g (34,9 mmol) (all-E)-Retinol und 545 mg (3,49 mmol) 2,2,6,6-Tetramethylpiperidin-1-oxyl in 75 ml N,N-Dimethylformamid leitete man nach Zugabe von 345 mg (3,49 mmol) Kupfer(I)chlorid bei Raumtemperatur 50 bis 60 ml Sauerstoff pro Minute. Nach zwei Stunden wurde auf 150 ml wässrige Natriumchlorid-Lösung gegossen und mit Methyl-tert.-butylether extrahiert, bis die Extraktionslösung farblos war. Man wusch mit Wasser, trocknete mit Natriumsulfat und entfernte das Lösungsmittel im Vakuum. Der Rückstand wurde an Kieselgel mittels Methylenchlorid schnell chromatographiert. Man erhielt 9,1 g (91,6 % Ausbeute) $^{1}$H-NMR-reines Produkt mit weniger als 5 % cis-Isomer. Kristallisation aus n-Hexan lieferte 7,2 g Retinal (72,6 % Ausbeute) mit einem Schmelzpunkt von 60 bis 62 °C und einem UV-Wert $E_1^1$ = 1515 bei 381 nm (Ethanol).

Beispiel 2

Oxidation von Retinol zu Retinal unter Verwendung von 4-Oxo-2,2,6,6-Tetramethylpiperidin-1-oxyl/Kupfer(I)-chlorid

Durch eine Lösung von 10,0 g (34,9 mmol) (all-E)-Retinol und 594 mg (3,49 mmol) 4-Oxo-2,2,6,6-Tetramethylpiperidin-1-oxyl in 75 ml N,N-Dimethylformamid leitete man nach Zugabe von 345 mg (3,49 mmol) Kupfer(I)chlorid bei Raumtemperatur 50 bis 60 ml Sauerstoff pro Minute. Nach drei Stunden wurde auf 150 ml wässrige Natriumchlorid-Lösung gegossen und mit Methyl-tert.-butylether extrahiert, bis die Extraktionslösung farblos war. Man wusch mit Wasser, trocknete mit Natriumsulfat und entfernte das Lösungsmittel im Vakuum. Der Rückstand wurde an Kieselgel mittels Cyclohexan/Essigester (2:1) schnell chromatographiert. Man erhielt 8,8 g (88,7 % Ausbeute) $^{1}$H-NMR-reines Produkt mit weniger als 5 % cis-Isomer, Kristallisation aus n-Hexan lieferte 6,9 g Retinal (69,5 % Ausbeute) mit einem Schmelzpunkt von 60 bis 62 °C und einem UV-Wert $E_1^1$ = 1520 bei 381 nm (Ethanol).

Beispiel 3

Oxidation von 8-Hydroxy-2,6-dimethyl-octa-2,4,6-trien-1-säureethylester mit 2,2,6,6-Tetramethylpiperidin-1-oxyl/Kupfer(I)chlorid

Durch eine Lösung von 30,0 g (142,7 mmol) 8-Hydroxy-2,6-dimethyl-octa-2,4,6-trien-1-säureethylester (Isomerenmischung) und 2,23 g (14,3 mmol) 2,2,6,6-Tetramethylpiperidin-1-oxyl in 75 ml N,N-Dimethylformamid leitete man nach Zugabe von 1,42 g (14,3 mmol) Kupfer(I)chlorid 50 bis 60 ml Sauerstoff pro Minute, wobei mit Hilfe eines Eisbades die Temperatur in einem Bereich von 20 bis 35 °C gehalten wurde. Nach einer Stunde goß man auf 150 ml wässrige Natriumchlorid-Lösung und extrahierte mit Methyl-tert.-butylether. Die organische Phase wurde zunächst mit einer Lösung von Natriumjodid in 5 proz. Salzsäure

und anschließend mit wässiger Natriumthiosulfat-Lösung geschüttelt. Nach Waschen mit Wasser und Trocknen über Natriumsulfat wurde das Lösungsmittel im Vakuum entfernt. Man erhielt 27,3 g (92 % Ausbeute) 2,6-Dimethyl-8-oxo-octa-2,4,6-trien-1-säureethylester.

Beispiel 4

Oxidation von 8-Hydroxy-2,5-dimethyl-octa-2,4,6-trien-1-al

Durch eine gerührte Lösung von 13,95 g (83,9 mmol) (all-E)-8-Hydroxy-2,6-dimethyl-octa-2,4,6-trien-1-al und 655 mg (4,2 mmol) 2,2,6,6-Tetramethylpiperidin-1-oxyl in 50 ml N,N-Dimethylformamid wurden nach Zugabe von 415 mg (4,2 mmol) Kupfer(I)chlorid bei 25 bis 35 °C 50 bis 60 ml Sauerstoff pro Minute geleitet.

Nach 3 Stunden goß man die Reaktionsmischung auf die doppelte Menge wäßriger Natriumchlorid-Lösung, extrahierte mit Methyl-tert.-butylether, wusch mit Wasser und trocknete. Beim Entfernen des Lösungsmittels im Vakuum verblieben 11,25 g (81,6 % Rohausbeute) (all-E)-2,6-Dimethyl-octa-2,4,6-trien-1,8-dial, welche einer schnellen Chromatographie an Kieselgel mittels Cyclohexan/Essigester (2:1) unterworfen wurden. Man erhielt 9,80 g (71,0 % Ausbeute) der gewünschten Verbindung.

$^1$H-NMR (200 MHz, $CDCl_3$): $\delta$ = 10.20 (d, 1H, J = 7.7 Hz), 9.57 (s, 1H), 7.20 (dd, 1H, J = 15.4 Hz, 10.8 Hz), 6.98 (dd, 1H, J = 10.8 Hz, 1.4 Hz), 6.78 (d, 1H, J = 15.4 Hz), 6.12 (d, 1H, J = 7.7 Hz), 2.40 (s, 3H), 1.98 (s, 3H)

Beispiel 5

Oxidation von 3-Methyl-7-(5,5-dimethyl-1,3-dioxan-2-yl)-octa-2,4,6-trien-1-ol

Durch eine gerührte Lösung von 10,0 g (39,6 mmol) (all-E)-3-Methyl-7-(5,5-dimethyl-1,3-dioxan-2-yl)-octa-2,4,6-trien-1-ol und 310 mg (1,98 mmol) 2,2,6,6-Tetramethylpiperidin-1-oxyl in 50 ml N,N-Dimethylformamid wurden nach Zugabe von 196 mg (1,98 mmol) Kupfer(I)chlorid bei 25 bis 35 °C 50 bis 60 ml Sauerstoff pro Minute geleitet.

Nach 7 Stunden goß man die Reaktionsmischung auf die doppelte Menge wäßriger Natriumchlorid-Lösung und extrahierte mit Methyl-tert.-butylether. Die organische Phase wurde getrocknet und das Lösungsmittel im Vakuum entfernt, wobei 8,8 g (88,7 % Rohausbeute) (all-E)-3-Methyl-7-(5,5-dimethyl-1,3-dioxan-2-yl)-octa-2,4,6-trien-1-al erhalten wurden. Kristallisation aus n-Hexan lieferte 6,9 g des gewünschten Aldehyds (69,6 % Ausbeute).

$^1$H-NMR (200 MHz, $CDCl_3$): $\delta$ = 10.15 (d, 1H, J = 7.8 Hz), 7.00 (dd, 1H, J = 15.7 Hz, 11.0 Hz), 6.40 (d, 1H, J = 15.7 Hz), 6.35 (d, 1H, J = 11.0 Hz), 5.98 (d, 1H, J = 7.8 Hz), 4.80 (s, 1H), 3.70 (d, 1H), 3.52 (d, 1H), 2.32 (s, 3H), 1.93 (s, 3H), 1.23 (s, 3H), 0.77 (s, 3H).

## Patentansprüche

**1.** Verfahren zur Herstellung von Polyenaldehyden der allgemeinen Formel I

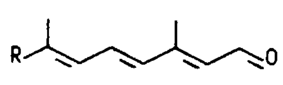 (I)

worin R für

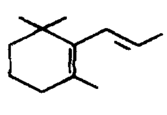 , 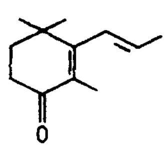 , 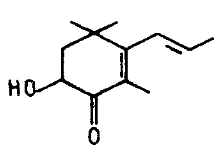 , 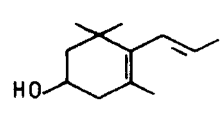 ,

-COOR' , -CHO oder

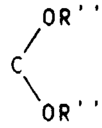

steht
wobei R′ die Bedeutung Alkyl, Aryl, Aralkyl, Cycloalkyl besitzt und gewünschtenfalls weiter substituiert sein kann und die Reste R″ für $C_1$-$C_4$-Alkyl stehen oder gemeinsam $-CH_2-CH_2-$ oder $-CH_2-CH_2-CH_2-$ Brücken bedeuten, die gewünschtenfalls durch $C_1$-$C_4$-Alkyl weiter substituiert sein können,
durch katalytische Oxidation des entsprechenden Polyenalkohols mit Sauerstoff oder einem sauerstoffhaltigen Gas, dadurch gekennzeichnet, daß man die Oxidation in Gegenwart des Katalysatorsystems 2,2,6,6-Tetramethylpiperidin-1-oxyl oder 4-Oxo-2,2,6,6-tetramethylpiperidin-1-oxyl und Kupfer(I)chlorid durchführt.

2. Verfahren nach Anspruch 1, dadurch gekennzeichnet, daß man (all-E)-Retinal aus (all-E)-Retinol herstellt.

3. Verfahren nach einem der Ansprüche 1 oder 2, dadurch gekennzeichnet, daß man in homogener Phase in einem Lösungsmittel arbeitet.

4. Verfahren nach Anspruch 3, dadurch gekennzeichnet, daß man als Lösungsmittel N,N-Dimethylformamid, N,N-Dimethylacetamid, 1-Methyl-2-pyrrolidon, N,N-Dimethylpropylenharnstoff oder N,N-Dimethylethylenharnstoff verwendet.

5. Verfahren nach einem der Ansprüche 1 bis 4, dadurch gekennzeichnet, daß man die Katalysatorkomponenten 2,2,6,6-Tetramethylpiperidin-1-oxyl oder 4-Oxo-2,2,6,6-tetramethylpiperidin-1-oxyl zu Kupfer(I)-chlorid im molaren Verhältnis von 1:0,5 bis 1:3 einsetzt.

6. Verfahren nach einem der Ansprüche 1 bis 5, dadurch gekennzeichnet, daß die einzelnen Katalysatorkomponenten jeweils in einem Anteil von 1 bis 10 mol%, insbesondere 5 bis 10 mol%, bezogen auf den eingesetzten Polyenalkohol, vorliegen.

**Claims**

1. A process for the preparation of a polyene aldehyde of the formula I

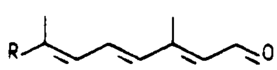 (I)

where R is

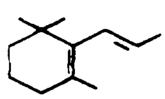 , 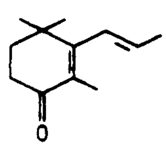 , 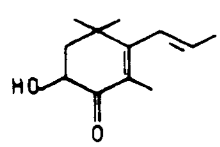 , 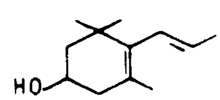

-COOR' , -CHO or

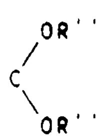

where R' is alkyl, aryl, aralkyl or cycloalkyl and, if desired, may be further substituted, and the radicals R'' are each $C_1$-$C_4$-alkyl or together form $-CH_2-CH_2$ or $-CH_2-CH_2-CH_2-$ bridges which, if desired, may be further substituted by $C_1$-$C_4$-alkyl, by catalytic oxidation of the corresponding polyene alcohol with oxygen or an oxygen-containing gas, wherein the oxidation is carried out in the presence of the catalyst system comprising 2,2,6,6-tetramethylpiperidine 1-oxide or 4-oxo-2,2,6,6,-tetramethylpiperidine 1-oxide and copper (I) chloride.

**2.** A process as claimed in claim 1, wherein (all-E)-retinal is prepared from (all-E)-retinol.

**3.** A process as claimed in claim 1 or 2, wherein the reaction is carried out in the homogeneous phase in a solvent.

**4.** A process as claimed in claim 3, wherein the solvent used is N,N-dimethylformamide, N,N-dimethylacetamide, 1-methyl-2-pyrrolidine, N,N-dimethylpropyleneurea or N,N-dimethylethyleneurea.

**5.** A process as claimed in any of claims 1 to 4, wherein the catalyst components 2,2,6,6,-tetramethylpiperidine 1-oxide or 4-oxo-2,2,6,6-tetramethylpiperidine 1-oxide and copper(I) chloride are used in a molar ratio of from 1:0.5 to 1:3.

**6.** A process as claimed in any of claims 1 to 5, wherein the individual catalyst components are each present in an amount of from 1 to 10, in particular from 5 to 10, mol %, based on the polyene alcohol used.

## Revendications

**1.** Procédé de préparation d'aldéhydes polyéniques de formule générale I

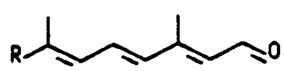 (I)

dans laquelle R est mis pour

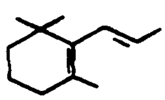 , 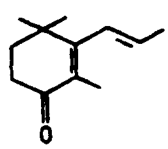 , 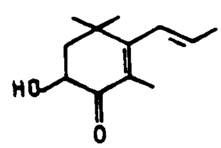 , 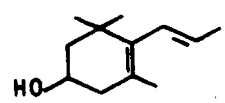

-COOR' , -CHO ou

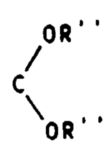

R' représentant un reste alkyle, aryle, aralkyle qui peut être au besoin substitué a son tour, et les restes R'' étant mis pour des radicaux alkyle en $C_1$-$C_4$ ou formant ensemble des ponts $-CH_2-CH_2-$ ou $-CH_2-CH_2-CH_2-$, qui peuvent être au besoin substitués à leur tour par des restes alkyle en $C_1$-$C_4$,
par oxydation catalytique de l'alcool polyénique correspondant par de l'oxygène ou un gaz contenant de l'oxygène, caractérisé en ce qu'on conduit l'oxydation en présence du système catalytique composé de 2,2,6,6-tétraméthylpipéridine-1-oxyde ou de 4-oxo-2,2,6,6-tétraméthylpipéridine-1-oxyde et de chlorure cuivreux.

**2.** Procédé selon la revendication 1, caractérisé en ce qu'on prépare du (tout-E)-rétinal à partir de (tout-E)-rétinol.

3. Procédé selon la revendication 1 ou 2, caractérisé en ce qu'on opère en phase homogène dans un solvant.

4. Procédé selon la revendication 3, caractérisé en ce qu'on utilise, comme solvant, du N,N-diméthylformamide, du N,N-diméthylacétamide, de la 1-méthyl-2-pyrrolidone, de la N,N-diméthylpropylène-urée ou de la N,N-diméthyléthylène-urée.

5. Procédé selon l'une quelconque des revendications 1 à 4, caractérisé en ce qu'on utilise les composants du catalyseur dans un rapport molaire du 2,2,6,6-tétraméthylpipéridine-1-oxyde ou du 4-oxo-2,2,6,6-tétraméthylpipéridine-1-oxyde au chlorure cuivreux de 1:0,5 à 1:3..

6. Procédé selon l'une quelconque des revendications 1 à 5, caractérisé en ce que les composants individuels du catalyseur sont présents chacun dans une proportion de 1 à 10% en moles, en particulier de 5 à 10% en moles, par rapport à l'alcool polyénique mis en réaction.